(19) Europäisches Patentamt
European Patent Office
Office européen des brevets

(11) Veröffentlichungsnummer: **0 052 800**
**B2**

(12) # NEUE EUROPÄISCHE PATENTSCHRIFT

(45) Veröffentlichungstag der neuen Patentschrift: **16.09.87**

(51) Int. Cl.⁴: **C 07 D 251/34**, C 09 J 3/14

(21) Anmeldenummer: **81109209.7**

(22) Anmeldetag: **29.10.81**

(54) **Acrylsäureester bzw. Methacrylsäureester von OH-gruppenhaltigen Isocyanursäurederivaten, Verfahren zu deren Herstellung sowie deren Verwendung als Klebstoff.**

(30) Priorität: **25.11.80 DE 3044318**

(43) Veröffentlichungstag der Anmeldung: **02.06.82 Patentblatt 82/22**

(45) Bekanntmachung des Hinweises auf die Patenterteilung: **22.08.84 Patentblatt 84/34**

(45) Bekanntmachung des Hinweises auf die Entscheidung über den Einspruch: **16.09.87 Patentblatt 87/38**

(84) Benannte Vertragsstaaten: **AT BE CH DE FR GB IT LI NL SE**

(56) Entgegenhaltungen:
**DE - A - 1 695 171**
**DE - A - 2 009 781**
**DE - A - 2 150 139**
**DE - A - 2 633 685**
**US - A - 3 932 401**

**Derwent Referat Nr. 87444 Y/49 der JP-A-52 128 387**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Gruber, Werner, Dr., Franz-Kremer-Strasse 7, D-4052 Korschenbroich (DE)**



ACTORUM AG

**Beschreibung**

Es ist bekannt, dass man die reaktionsfähigen Epoxidgruppen des kristallisierten Triglycidylisocyanurats mit aciden Wasserstoff enthaltenden Verbindungen wie Carbonsäuren und dergleichen reagieren lassen kann. Dabei werden aus dem hochschmelzenden kristallisierten Triglycidylisocyanurat harzartige Verbindungen erhalten, die sich zusammen mit Härtern (Vernetzern) für Epoxidharze aufgrund des hohen Gehaltes an nicht umgesetzten Glycidylgruppen zu Formkörpern und ähnlichen verarbeiten lassen (DE-A Nr. 1 695 171).

Aufgabe der vorliegenden Erfindung war es aber, neue definierte, polymerisationsfähige Isocyanuratverbindungen herzustellen, die frei sind von Epoxidgruppen und nicht aus Triglycidylisocyanurat hergestellt werden. Eine weitere Aufgabe bestand darin, eine Gruppe von Klebstoffen und Dichtungsmassen auf Basis neuartiger polymerisationsfähiger Verbindungen zu finden. Gegenstand der vorliegenden Erfindung ist die Verwendung der Verbindungen der allgemeinen Formel (I):

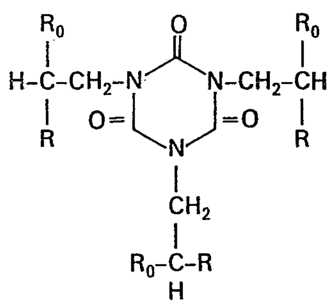

(I)

wobei $R_0$ = H oder $-CH_3$ oder $-C_2H_3$ und R =

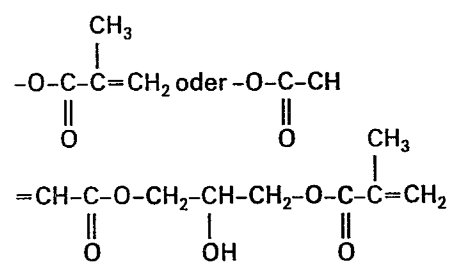

sein kann mit der Massgabe, dass eine von drei R-Gruppen auch -OH sein kann sowie anstelle einer Methacrylestergruppe auch eine Acrylestergruppe stehen kann beziehungsweise der Gemische der Verbindungen als wesentlicher Bestandteil von in an sich bekannter Weise Peroxide als Initiator enthaltenden bei Sauerstoffausschluss erhärtenden Klebstoffen beziehungsweise Dichtungsmitteln für metallische Flächen.

Es ist bekannt, dass man aus Cyanursäure und Alkylenoxiden leicht die entsprechenden Isocyanursäureester herstellen kann, wobei in alpha-Stellung eine reaktionsfähige Hydroxylgruppe entsteht:

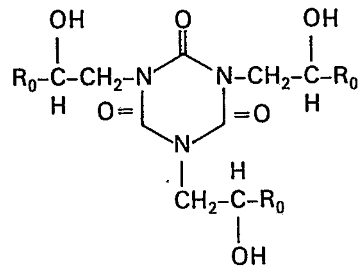

Je nach eingesetztem Alkylenoxid kann der Rest $R_0$ verschieden sein. Im vorliegenden Fall ist er entweder Wasserstoff oder Methyl oder Ethyl. Man geht also zur Herstellung der erfindungsgemässen Verbindungen aus von Trihydroxyalkyl-$(C_2-C_4)$isocyanursäureestern.

Das Verfahren zur Herstellung der Substanzen geht von den im vorstehenden Formelbild wiedergegebenen Strukturen aus. Dazu werden Trihydroxyalkylisocyanursäureester

a) in an sich bekannter Weise in (Meth)acrylester überführt, oder nach einer erfindungsgemässen Ausführungsform zur Herstellung der neuen Verbindungen gemäss Anspruch 2,

b) mit Maleinsäureanhydrid in die freie Carboxylgruppen aufweisenden Halbester überführt und dann diese mit Glycidyl(meth)acrylat umgesetzt. Nach der erfindungsgemässen Verwendung nach Anspruch 1 können dabei gegebenenfalls bis ⅓, berechnet auf molare Verhältnisse, an Carboxylgruppen unverestert bleiben.

Zur Verdeutlichung der notwendigen Reaktionsschritte wird zusätzlich noch im einzelnen das Folgende bemerkt.

Ausgehend von den vorstehend genannten Verbindungen kann man entweder die OH-Gruppen direkt mit Methacrylsäure unter den an sich bekannten Bedingungen verestern oder mit Methacrylsäurechlorid in Gegenwart geeigneter Lösungsmittel wie Dimethylformamid oder dergleichen den Methacrylsäurerest einführen. Wenn partiell der Acrylsäurerest eingeführt werden soll, kann dies durch entsprechenden Ersatz von Methacrylsäure beziehungsweise dessen Chlorid durch Acrylsäure beziehungsweise dessen Chlorid geschehen.

Weiterhin kann man ausgehend von den vorstehend genannten Verbindungen die OH-Gruppen mit Maleinsäureanhydrid in die freie Carboxylgruppen aufweisenden Halbester überführen. Diese werden dann mit Glycidylmethacrylat, welches bis zu ⅓ durch Glycidylacrylat ersetzt sein kann, zu den Tri(meth)acrylsäureestern umgesetzt. Verwendet man – berechnet auf molare Verhältnisse – bis zu ⅓ weniger an Glycidyl(meth)acrylat, so erhält man noch freie Carboxylgruppen aufweisende Verbindungen, die im wesentlichen aus Di(meth)acrylsäureesterderivaten der Trihydroxyalkylisocyanursäure bestehen.

Die erfindungsgemässen Methacrylsäureester sind Monomere, die mittels bekannter Initiatoren

in hochmolekulare Verbindungen überführt werden können. Sie können vorteilhaft verwendet werden als Bestandteil von bei Sauerstoffausschluss erhärtenden Acrylsäureesterklebstoffen. Bei Verwendung der erfindungsgemässen Ester werden besonders warmfeste Verklebungen erhalten. Sie sind Klebemischungen auf Basis von Dimethacrylsäureestern aus propoxyliertem Diphenylolpropan deutlich überlegen.

Die erfindungsgemässen Klebstoffe können auch bis zu 50 Gew.% weitere polymerisierbare Anteile, z.B. Monomethacrylate wie Tetrahydrofurfurylmethacrylat, 5,6-Dihydrodicyclopentadienylmethacrylat, Cyclohexylmethacrylat, Ethylhexylmethacrylat, Hydroxyethylmethacrylat, Hydroxypropylmethacrylat oder Dimethacrylate wie Ethylenglykoldimethacrylat, Triethylenglykoldimethacrylat, Polyethylenglykoldimethacrylat enthalten.

Als Initiator enthalten die Klebstoffe und Dichtungsmassen Peroxide, insbesondere Hydroperoxide, wie Cumolhydroperoxid oder tert.-Butylhydroperoxid. Diese werden in der Regel in Mengen von 1 bis 10 Gew.% auf polymerisierbare Anteile zugesetzt.

Zweckmässigerweise werden den Klebstoffen und Dichtungsmitteln Stabilisatoren und Beschleuniger zugesetzt. Als Stabilisatoren eignen sich Chinon oder Hydrochinon in Konzentrationen von 100 bis 1000, vorzugsweise 200 bis 500 ppm, bezogen auf polymerisierbare Anteile. Als Beschleuniger sind sogenannte Imidbeschleuniger, wie Benzoesäuresulfimid, insbesondere aber Sulfohydrazidbeschleuniger, wie p-Toluolsulfonsäurehydrazid, in Kombination mit einem tert.-Amin, vorzugsweise N,N-Dimethyl-p-toluidin, geeignet. Beschleuniger und Stabilisator müssen in aufeinander abgestimmten Verhältnissen zugesetzt werden, um optimale Eigenschaften der Klebstoffe oder Dichtungsmittel zu erzielen. Im allgemeinen werden sie in Mengen von 0,1 bis 3 Gew.%, bezogen auf polymerisierbare Anteile, eingesetzt.

Für bestimmte Anwendungszwecke können diese Klebstoffe oder Dichtungsmassen Zusätze an Weichmachern, Verdickungsmitteln oder Farbstoffen enthalten. Als Verdickungsmittel zur Erhöhung der Viskosität eignen sich insbesondere Polymerisate des Methacrylsäuremethylesters.

Die Klebstoffe oder Dichtungsmassen werden durch Mischen der Komponenten bei Raumtemperatur hergestellt. Sie sind in den meisten Fällen jahrelang stabil, sofern sie in luftdurchlässigen Gefässen, wie Polyethylenflaschen, gelagert werden. Bringt man die Klebstoffe oder Dichtungsmittel zwischen Metalloberflächen, so polymerisieren sie rasch unter Bildung einer festen Verbindung der Flächen. Die Vorteile der erfindungsgemäss erhältlichen Klebstoffe oder Dichtungsmittel sind unter anderem darin zu sehen, dass die zu verbindenden Teile bei Raumtemperatur verklebbar und schon nach kurzer Zeit belastbar sind.

Die Wärmefestigkeit der Klebeverbindungen ist in der Regel hervorragend. Demnach sind die erfindungsgemässen Klebstoffe zum Verbinden von Metallen besonders dann geeignet, wenn hohe Festigkeit bei guter Wärmestabilität der Klebefuge gefordert wird. Sie finden daher in der Technik Anwendung zum Verkleben von Blechen beziehungsweise Werkstoffen aus verschiedenen Metallen, zur Befestigung von Lagerwellen, zum Abdichten von Rohrverbindungen und dergleichen mehr. Auffällig ist der relativ schwache Abfall der Festigkeit zwischen 100 und 150°C.

Selbst bei 200°C sind noch fast 50% des bei Zimmertemperatur gemessenen Drehkraftmoments zu beobachten.

Beispiel 1

Trismethacryloylethylisocyanurat

175 g (0,67 mol) Trishydroxyethylisocyanursäureester wurden in 200 ml Dimethylformamid gelöst und in Gegenwart von 202 g (2 mol) Triethylamin mit 208 g (2 mol) Methacrylsäurechlorid bei ~5°C umgesetzt. Anschliessend wurde 1 h auf 50°C erwärmt. Der Ansatz wurde in Wasser gegossen und der Trimethacrylsäureester in Methylenchlorid aufgenommen. Nach dem Trocknen und Abdampfen des Lösungsmittels hinterblieb ein gelbbraunes viskoses Öl.

Ausbeute: 250 g (81% d.Th.)

| Analyse: | | | |
|---|---|---|---|
| Berechnet: | C 54,19 | H 5,81 | N 9,03% |
| Gefunden: | C 54,00 | H 5,35 | N 9,23% |

Fp.: 65°C.

Beispiel 2

Bismethacryloylethylhydroxyethylisocyanurat

175 g (0,67 mol) Trishydroxyethylisocyanursäureester wurden in 200 ml Dimethylformamid gelöst und in Gegenwart von 131 g (1,3 mol) Triethylamin und 135 g (1,3 mol) Methacrylsäurechlorid bei ~5°C umgesetzt. Die Aufarbeitung erfolgte wie im Beispiel 1 beschrieben.

Ausbeute: 240 g (80% d.Th.)

| Analyse: | | | |
|---|---|---|---|
| Berechnet: | C 51,39 | H 5,79 | N 10,58% |
| Gefunden: | C 51,70 | H 6,16 | N 10,20% |

$n_D^{20}$: 1,5107

Beispiel 3

Trimethacrylsäureester des Maleinathalbesters von Trishydroxyethylisocyanurat

392 g (1,5 mol) Trishydroxyethylisocyanursäureester und 441 g (4,5 mol) Maleinsäureanhydrid wurden bei 100°C so lange zur Reaktion gebracht, bis die Säurezahl bei 315 (ber. 302) lag. Anschliessend wurde bei 80°C mit 639 g (4,5 mol) Glycidylmethacrylsäureester umgesetzt. Nach 10 h lag die Säurezahl bei 10.

Ausbeute: 1472 g (100% d.Th.)

| Analyse: | | | |
|---|---|---|---|
| Berechnet: | C 51,06 | H 5,78 | N 4,26% |
| Gefunden: | C 50,50 | H 5,37 | N 4,30% |

$n_D^{20}$: 1,521

Beispiel 4
Dimethacrylsäureester des Maleinathalbesters von Trishydroxyethylisocyanurat

Der Halbester wurde - wie im Beispiel 3 beschrieben - hergestellt. Die Methacryloylierung erfolgte bei 80°C mit 425 g (3 mol) Glycidylmethacrylsäureester bis zur Säurezahl von 110 (ber. 101).

Ausbeute: 1258 g (100% d.Th.)

| Analyse: | | | |
|---|---|---|---|
| Berechnet: | C 49,93 | H 5,77 | N 5,64% |
| Gefunden: | C 50,00 | H 5,28 | N 5,23% |

$n_D^{20}$: 1,533

Beispiel 5
Klebemischungen aus Di- und Trimethacrylatsäureestern

Je 50 Teile der Di- oder Trimethacrylsäureester der Beispiele 1 bis 4 wurden mit 20 g Hydroxyethylmethacrylsäureester und 20 g Triethylenglykoldimethacrylsäureester verdünnt. Diese Monomerengemische wurden mit 5 g Polymethylmethacrylsäureester, 1 g p-Toluolsulfonsäurehydrazid, 3 g Cumolhydroperoxid und 1 g Triethylamin versetzt. Die resultierenden anaeroben Klebstoffe wurden hinsichtlich ihrer Eigenschaften überprüft. Die Stabilität, gemessen im üblichen 80°C-Tempertest, lag bei allen Mischungen oberhalb von 30 min. Dazu wurde ein 10 cm langes und 10 mm weites Reagenzglas zu 9/10 mit der Mischung gemäss Beispielen 1 bis 4 gefüllt, in ein auf 80°C gehaltenes Bad eingehängt und bis zur ersten Gelbildung beobachtet. Von einer Fortsetzung der beschleunigten Alterung wurde abgesehen, da diese Prüfung bedeutet, dass die Produkte bei Raumtemperatur etwa ein Jahr unverändert haltbar sind.

Zur Prüfung der Drehkraftmomente wurden Schrauben (M 10×30 DIN 933-8.8) und Muttern (M 10 DIN 934-5.6) verklebt und nach dreitägiger Aushärtung bei Raumtemperatur in einen Schraubstock eingespannt und mit einem Drehmomentschlüssel das Drehkraftmoment bestimmt. Die Druckscherfestigkeit wurde an verklebten Buchsen aus Stahl ST 50 K (Höhe 10 mm, Durchmesser 20 mm) und Bolzen aus Stahl ST 50 K (Höhe 10 mm, Durchmesser 19,85 mm) nach Lagerung (24 h) bei Raumtemperatur bestimmt (DIN-Entwurf 54 452).

In einem weiteren Test wurde die Druckscherfestigkeit nach 1 d an 3 h auf 180°C erwärmten Prüfkörpern bestimmt. Das Aufheizen erfolgte in einem Wärmeschrank während der angegebenen Zeit, die Prüfung unmittelbar nach der Entnahme aus dem Wärmeschrank.

Alle Tests wurden 5mal durchgeführt und dann der Mittelwert der Prüfungsergebnisse angegeben. In der nachfolgenden Tabelle sind in Abhängigkeit von den Herstellungsbeispielen die ermittelten Werte wiedergegeben.

Tabelle

| Klebmischung gemäss Beispiel Nr. | Drehmoment in Nm nach 3 h | Druckscherfestigkeit in N/mm² nach 24 h | Druckscherfestigkeit in N/mm² nach 24 h 20°C und 3 h 180°C |
|---|---|---|---|
| 1 | 30 | 33 | 25 |
| 2 | 35 | 35 | 25 |
| 3 | 50 | 33 | 15 |
| 4 | 40 | 31 | 15 |

Vergleichsversuch

Der Dimethacrylsäureester des Umsetzungsprodukts von 2 mol Propylenoxid und 1 mol Diphenylolpropan wurde - wie in dem vorstehenden Beispiel 5 beschrieben - mit den angegebenen Hilfsstoffen versetzt. Dann wurden die dort beschriebenen Tests vorgenommen.

Druckscherfestigkeit nach 24 h: 35 N/mm²
Druckscherfestigkeit bei 180°C: 10 N/mm².

**Patentansprüche**

1. Verwendung der Verbindungen der allgemeinen Formel (I):

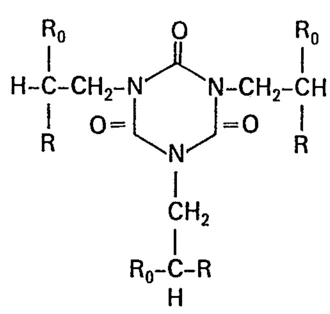

(I)

wobei $R_0$ = H oder $-CH_3$ oder $-C_2H_5$ und R =

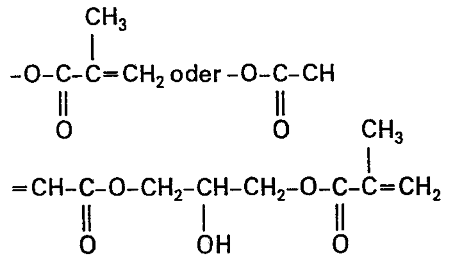

sein kann mit der Massgabe, dass eine von drei R-Gruppen auch -OH sein kann sowie anstelle einer Methacrylestergruppe auch eine Acrylestergruppe stehen kann beziehungsweise der Gemische der Verbindungen als wesentlicher Bestandteil von in an sich bekannter Weise Peroxide als Initiator enthaltenden bei Sauerstoffausschluss erhärtenden Klebstoffen beziehungsweise Dichtungsmitteln für metallische Flächen.

2. Isocyanursäureester der allgemeinen Formel (Ia):

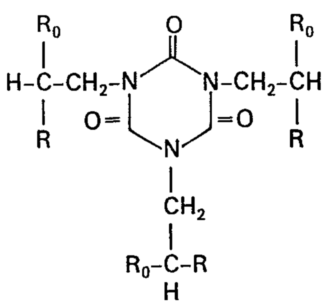

(Ia)

wobei $R_0$ = H oder $-CH_3$ oder $-C_2H_5$ und R =

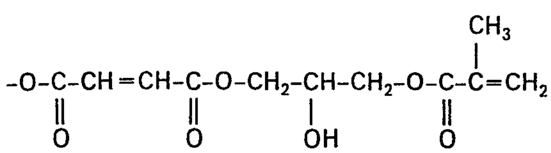

sein kann mit der Massgabe, dass anstelle einer Methacrylestergruppe auch eine Acrylestergruppe stehen kann.

3. Verfahren zur Herstellung von Isocyanursäureestern gemäss Anspruch 2, dadurch gekennzeichnet, dass man Isocyanursäurederivate der allgemeinen Formel (II):

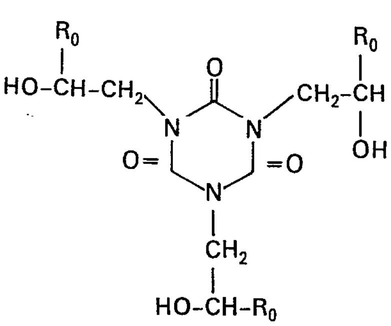

(II)

wobei $R_0$ = H oder $-CH_3$ oder $-C_2H_5$ und R =

mit Maleinsäureanhydrid in die freie Carboxylgruppen aufweisenden Halbester überführt und dann diese mit Glycidyl(meth)acrylat umsetzt.

**Claims**

1. The use of compounds corresponding to the following general formula

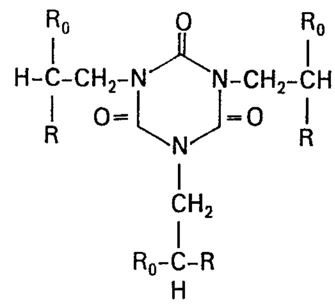

(I)

in which $R_0$ = H or $-CH_3$ or $-C_2H_5$ and R =

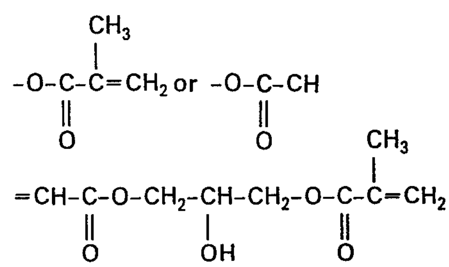

with the proviso that one of three R groups may also be -OH and a methacrylic ester group may be replaced by an acrylic ester group, or mixtures of these compounds as an essential constituent of adhesives or sealing compounds for metallic surfaces which contain peroxides as initiator and which set in the absence of oxygen.

2. A process for the production of isocyanuric acid esters, characterized in that isocyanuric acid derivatives corresponding to the following general formula

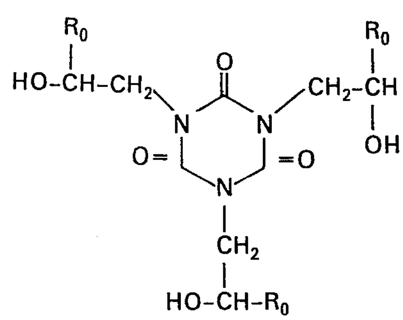

(II)

in which $R_0$ = H or $-CH_3$ or $-C_2H_5$ and R =

are converted with maleic acid anhydride into the semi-esters containing free carboxyl groups

which are then reacted with glycidyl(meth)acrylate.

3. Isocyanuric acid esters corresponding to the following general formula

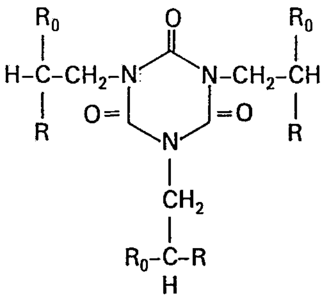

(I)

in which $R_o$ = H or $-CH_3$ or $-C_2H_5$ and R =

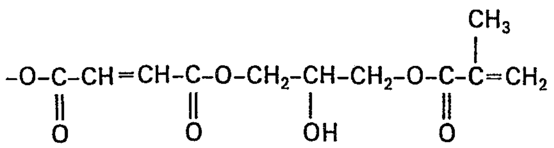

with the proviso that a methacrylic ester group may be replaced by an acrylic ester group.

**Revendications**

1. Utilisation des composés de formule générale (I):

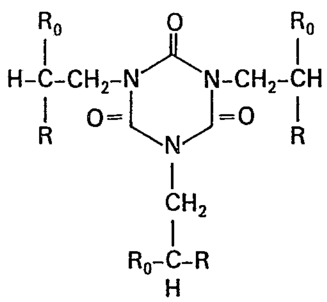

(I)

dans laquelle $R_o$ peut être H ou $-CH_3$ ou $-C_2H_5$ et R peut être

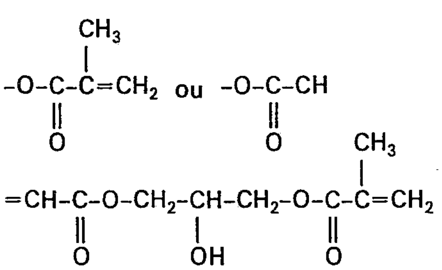

ou

avec la mesure qu'un des trois groupes R peut être aussi un -OH et qu'au lieu d'un groupe ester méthacrylique, on puisse aussi avoir un groupe ester acrylique, ou des mélanges de ces composés, comme constituant essentiel d'adhésifs ou d'agents d'étanchéification de surfaces métalliques, contenant, de façon connue en soi, des peroxydes en tant qu'initiateurs durcissant à l'abri de l'oxygène.

2. Procédé de préparation d'esters d'acide isocyanurique, caractérisé en ce que des dérivés d'acide cyanurique de formule générale (II):

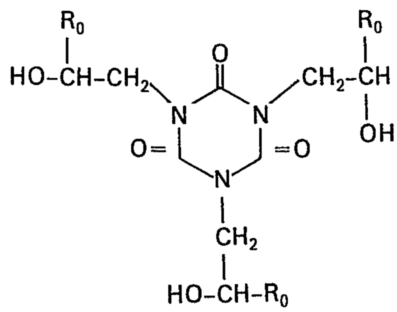

(II)

dans laquelle $R_o$ peut être H ou $-CH_3$ ou $-C_2H_5$, sont mis à réagir avec de l'anhydride maléique en semi-esters présentant des groupes carboxyle libres, lesquels sont alors mis à réagir avec du (meth)acrylate de glycidyle.

3. Esters d'acide isocyanurique de formule générale (I):

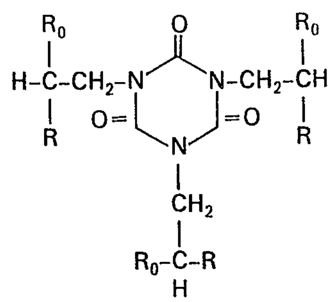

dans laquelle $R_o$ peut être H ou $-CH_3$ ou $-C_2H_5$ et R peut être

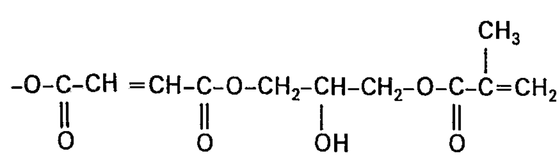

avec la mesure qu'au lieu d'un groupe ester méthacrylique, on puisse aussi avoir un groupe ester acrylique.